(19) Europäisches Patentamt
European Patent Office
Office européen des brevets

(11) Publication number: **0 139 005**
**A1**

(12) # EUROPEAN PATENT APPLICATION

published in accordance with Art. 158(3) EPC

(21) Application number: **84900104.5**

(51) Int. Cl.[4]: **A 61 K 31/27**

(22) Date of filing: **14.12.83**

Data of the international application taken as a basis:

(86) International application number:
**PCT/JP83/00437**

(87) International publication number:
**WO84/02272 (21.06.84 84/15)**

(30) Priority: **16.12.82 JP 221709/82**

(43) Date of publication of application:
**02.05.85 Bulletin 85/18**

(84) Designated Contracting States:
**BE DE FR GB NL SE**

(71) Applicant: **Yoshitomi Pharmaceutical Industries, Ltd.**
**35 Hiranomachi 3-chome Higashi-ku**
**Osaka-shi Osaka 541(JP)**

(72) Inventor: **HASEGAWA, Gen**
**42-1 Oaza-Kanate Nakatsu-shi**
**Oita 871(JP)**

(72) Inventor: **OKUMOTO, Takeki 1005 Higashinakano Heim**
**23-6, Higahsinakano 5-chome**
**Nakano-ku Tokyo 164(JP)**

(74) Representative: **von Kreisler, Alek, Dipl.-Chem. et al,**
**Deichmannhaus am Hauptbahnhof**
**D-5000 Köln 1(DE)**

(54) **ANTITUMOR COMPOSITION, METHOD FOR TREATING TUMOR, AND USE OF THE COMPOSITION FOR TREATING TUMOR.**

(57) S-methylcarbamoyl-L-cysteine ethyl ester represented by the formula: (I) wherein the α-carbon atom marked with an asterisk has an L configuration, and pharmaceutically acceptable acid addition salts thereof show an antitumor effect on various experimental tumors including human tumor models. The invention provides antitumor compositions containing them as effective ingredients, and method for treating tumors using the composition.

```
         O       O
         ‖   *   ‖
CH3NHCSCH2CHCOC2 H5        (I)
             |
            NH2
```

EP 0 139 005 A1

TITLE MODIFIED
see front page

SPECIFICATION

# Antitumor Compositions, Therapeutic Methods of Tumors and Use for Tumor-Therapy

## Technical Field and Disclosure of Invention

The present invention relates to antitumor compositions, therapeutic methods of tumors and use for therapy of tumor. It has been known that certain species of cysteine compounds have antitumor activity. For example, R.H. Adamson reported in Nature [217, 751-752 (1968)] that S-carbamoyl-L-cysteine was effective against leukemia in mice. It was also reported by L. Németh et al. in Arzneim.-Forsh./Drug Res. [28(II), Heft 7, 1119-1123 (1978)] that S-ethylcarbamoyl-L-cysteine (hereinafter called ESCC) showed more excellent effect than S-carbamoyl-L-cysteine against experimental tumors.

In view of recent remarkable increase of patients suffering from cancer, the present inventors have made extensive study for developing more effective antitumor agents to find that S-methylcarbamoyl-L-cysteine ethyl ester representable by the formula (I);

$$CH_3NH\overset{\overset{O}{\|}}{C}SCH_2\underset{\underset{NH_2}{|}}{\overset{*}{C}}H\overset{\overset{O}{\|}}{C}OC_2H_5 \qquad (I)$$

(wherein the carbon atom with asterisk at the α-position is L-configurated)

or a pharmaceutically acceptable acid addition salt thereof

is possessed of excellent effects against various experimental tumors, thus completing the present invention.

The compounds of this invention, uses thereof as antibacterial agents and a method of preparing them have been known as disclosed in Japanese Patent Publication (after examination) No.56-37223 (37223/1981) or Japanese Patent Publication (after examination) No.56-37224 (37224/1981), but the uses as antitumor agents have not been known. The compounds employed in the present invention can be prepared by allowing L-cysteine ethyl ester or an acid addition salt thereof to react with methyl isocyanate in an inert solvent (e.g. water, ethanol, acetone, benzene, tetrahydrofuran, ethyl acetate, dimethyl formamide or a mixture of two or more of them) and, when necessary, in the presence of a catalytic amount of a base, or by allowing S-methylcarbamoyl-L-cysteine or an acid addition salt thereof to react with ethanol in accordance with conventional method of esterification of amino acid. When the resulting product is a free base, it may be converted by a per se conventional method into a pharmaceutically acceptable acid addition salt (e.g. hydrochloride, hydrobromide, sulfate, acetate, methanesulfonate, paratoluenesulfonate, oxalate, maleate, succinate, tartrate, etc.).

The typical example of the compounds employed for the present invention is S-methylcarbamoyl-L-cysteine ethyl ester hydrochloride (hereinafter called Y-6430), which is obtained as colorless needles, m.p. 134-136°C.

Pharmacological effects and toxicities of compounds employed as effective components of the compositions of this

invention are described as follows:

Experimental Example 1

A plurality of groups each consisting of five heads of female $CDF_1$ mice (7-8 weeks old) were implanted intraperitoneally with $10^6$ cells of IMC carcinoma. After 24 hours, Y-6430 was respectively administered once daily for seven days, and average survival times, life-prolongation ratios and 30-day survivors were calculated. From the results shown in Table 1, it is revealed that Y-6430 administered orally or intraperitoneally was effective against IMC carcinoma.

Table 1

| Dose (mg/kg/day) | Administration Route | Average Survival Time (days) | Life-Prolongation Ratio (%) | 30-day Survivors |
|---|---|---|---|---|
| 100 | i.p. | 24.0 | 218 | 0/5 |
| 250 | | 25.0 | 227 | 1/5 |
| 500 | | 27.0 | 245 | 1/5 |
| 0 | | 11.0 | 100 | 0/5 |
| 100 | p.o. | 12.6 | 115 | 0/5 |
| 250 | | 18.2 | 165 | 0/5 |
| 500 | | 21.0 | 191 | 0/5 |
| 1,000 | | 23.0 | 209 | 0/5 |
| 0 | | 11.0 | 100 | 0/5 |

The life-prolongation ratio was calculated by the following scheme (the same scheme is applied to the subsequent experimental examples).

$$\text{Life-Prolongation Ratio (\%)} = \frac{T}{C} \times 100$$

T : Average survival days of the treated group

C : Average survival days of the control group

Animals survived over 30 days or longer were estimated as 30-day survivors, and the cases where T/C equals to or greater than 125% were regarded as effective.

Experimental Example 2

A plurality of groups each consisting of five heads of female $CDF_1$ mice (7-8 weeks old) were respectively implanted intraperitoneally with $10^6$ cells of P388 leukemia. After 24 hours, Y-6430 and ESCC were respectively administered once daily for seven days, and average survival days, life-prolongation ratios and 30-day survivors in each group were calculated. The results were shown in Table 2 and 3, from which it was revealed that Y-6430 was effective against P388 leukemia while ESCC was only slightly effective.

Table 2

| Dose of Y-6430 (mg/kg/day) | Administration Route | Average Survival Time (days) | Life-Prolongation Ratio (%) | 30-day Survivors |
|---|---|---|---|---|
| 100 | i.p. | 13.4 | 134 | 0/5 |
| 250 | | 14.6 | 146 | 0/5 |
| 500 | | 17.2 | 172 | 0/5 |
| 0 | | 10.0 | 100 | 0/5 |
| 100 | p.o. | 11.6 | 116 | 0/5 |
| 250 | | 13.4 | 134 | 0/5 |
| 500 | | 15.0 | 150 | 0/5 |
| 1,000 | | 16.6 | 166 | 0/5 |
| 0 | | 10.0 | 100 | 0/5 |

Table 3

| Dose of ESCC (mg/kg/day) | Administration Route | Average Survival Time (days) | Life-Prolongation Ratio (%) | 30-day Survivors |
|---|---|---|---|---|
| 100 | p.o. | 10.2 | 102 | 0/5 |
| 250 | | 10.8 | 108 | 0/5 |
| 500 | | 10.8 | 108 | 0/5 |
| 1,000 | | 12.6 | 126 | 0/5 |
| 0 | | 10.0 | 100 | 0/5 |

Experimental Example 3

A plurality of groups each consisting of five heads of female $CDF_1$ mice (8 weeks old) were respectively implanted intraperitoneally with $10^5$ cells of L1210 leukemia. After the next day of the implantation, Y-6430, ESCC and ESCC ethyl ester (hereinafter referred to ESCC-Et) in physiological saline were respectively administered once daily for seven days intraperitoneally, and the average survival days and life-prolongation ratios in each group were calculated. From the results shown in the Table 4, it was revealed that Y-6430 was effective in the group administered with 500 mg/kg while ESCC and ESCC-Et were ineffective.

Table 4

| Compound | Dose (mg/kg/day, i.p.) | Average Survival Time (days) | Life-Prolongation Ratio (%) |
|---|---|---|---|
| Y-6430 | 50 | 8.4 | 105 |
| | 100 | 9.4 | 118 |
| | 250 | 9.8 | 123 |
| | 500 | 12.4 | 155 |
| | 0 | 8.0 | 100 |
| ESCC | 50 | 8.0 | 108 |
| | 100 | 8.0 | 108 |
| | 250 | 9.0 | 122 |
| | 500 | 9.0 | 122 |
| | 0 | 7.4 | 100 |
| ESCC-Et | 50 | 8.2 | 103 |
| | 100 | 8.4 | 105 |
| | 250 | 9.0 | 113 |
| | 500 | 9.0 | 113 |
| | 0 | 8.0 | 100 |

All the test animals died of tumor within 30 days.

Experimental Example 4

A plurality of groups each consisting of five female $CDF_1$ mice (7-8 weeks old) were respectively implanted intraperitoneally with $10^5$ cells of L1210 leukemia. After the next day of the implantation, the test animals were administered orally or subcutaneously once daily for seven days with Y-6430 or ESCC dissolved in physiological saline, and the average survival days and life-prolongation ratios in each

group were calculated. From the results shown in Table 5, it was revealed that Y-6430 was effective both by oral and subcutaneous administration while ESCC was ineffective.

Experimental Examples 3 and 4 showed that Y-6430 was effective against leukemia L1210 which is considered to have a high predictive value for clinical use [Cancer Chemother. Rep., 50, 173-218 (1966), Cancer Chemother. Rep. Part 3, 4, 189-198 (1973)], but ESCC was ineffective.

Table 5

| Compound | Dose (mg/kg/day) | Administration Route | Average Survival Time (days) | Life-Prolongation Ratio (%) |
|---|---|---|---|---|
| Y-6430 | 250 | p.o. | 9.4 | 115 |
| | 500 | | 11.4 | 139 |
| | 1,000 | | 13.8 | 168 |
| | 0 | | 8.2 | 100 |
| | 100 | s.c. | 8.2 | 103 |
| | 250 | | 9.2 | 115 |
| | 500 | | 10.8 | 135 |
| | 0 | | 8.0 | 100 |
| ESCC | 500 | p.o. | 8.0 | 98 |
| | 1,000 | | 9.2 | 112 |
| | 2,000 | | 9.6 | 117 |
| | 0 | | 8.2 | 100 |
| | 100 | s.c. | 8.0 | 100 |
| | 250 | | 8.0 | 100 |
| | 500 | | 8.0 | 100 |
| | 0 | | 8.0 | 100 |

All the test animals died of tumor within 30 days.

Experimental Example 5

A plurality of groups each consisting of six male Donryu rats (7-8 weeks old) were respectively implanted intraperitoneally with $10^6$ cells of AH44 hepatoma. Three days after the implantation, each test animal was once administered orally or intraperitoneally with Y-6430. The Table 6 shows the average survival days and number of survivors on 30th day after the administration.

Table 6

| Dose (mg/kg) | Administration Route | Average Survival Time (days) | 30-day Survivors |
|---|---|---|---|
| 500 | p.o. | 11.2 | 0/6 |
| 1,000 | p.o. | 10.8 | 0/6 |
| 1,000 | i.p. | 14.7 | 1/6 |
| 0 | | 7.2 | 0/6 |

Experimental Example 6

A plurality of groups each consisting of six male ICR mice (7-8 weeks old) were implanted intraperitoneally with $2 \times 10^6$ Ehrlich carcinoma. After 24 hours, Y-6430 dissolved in physiological saline was administered intraperitoneally once daily for seven days. Average survival days and number of survivors after 30 days were calculated, the results being shown in Table 7.

Table 7

| Dose (mg/kg/day, i.p.) | Average Survival Time (days) | 30-day Survivors |
|---|---|---|
| 50 | 18.6 | 0/12 |
| 100 | 26.0 | 6/12 |
| 0 | 13.2 | 0/12 |

Experimental Example 7

A plurality of groups each consisting of six male ICR mice (7-8 weeks old) were respectively implanted subcutaneously with 5 x $10^6$ Ehrlich carcinoma. After 24 hours, Y-6430 dissolved in physiological saline was administered intraperitoneally once daily for seven days. 24 hours after the final administration, the test animals were killed by ether anesthesia, and the carcinoma of each test animal was exercised and weighed. Table 8 shows the average weights.

Table 8

| Dose (mg/kg/day, i.p.) | Average Tumor Weight (mg) |
|---|---|
| 50 | 194.0 |
| 100 | 146.6 |
| 0 | 399.5 |

Experimental Example 8

A plurality of groups each consisting of six male Donryu rats (7-8 weeks old) were implanted with 2 x $10^6$ cells of AH272 hepatoma. After the implantation, Y-6430 dissolved in physiological saline was administered intraperitoneally once

daily within the period from the third to sixth day, and the average survival days were calculated, the result being shown in Table 9.

Table 9

| Dose (mg/kg/day, i.p.) | Average Survival Time (days) |
|---|---|
| 100 | 10.5 |
| 250 | 15.0 |
| 0 | 7.9 |

All the test animals were dead within 30 days.

Experimental Example 9

A plurality of groups each consisting of 12 male ICR mice (6-8 weeks old) were implanted intraperitoneally with 2 x $10^6$ cells of sarcoma 180. After the next day to the implantation, Y-6430 was administered intraperitoneally or subcutaneously once daily for seven days. Number of dead test animals, average survival days and 30-day survivors are calculated and shown in Table 10.

Table 10

| Dose (mg/kg/day) | Administration Route | Number of Dead Animals | Average Survival Time (days) | 30-day Survivors |
|---|---|---|---|---|
| 50 | i.p. | 0/12 | 25.5 | 0/12 |
| 100 | | 0/12 | 27.7 | 5/12 |
| 50 | s.c. | 0/12 | 21.9 | 0/12 |
| 100 | | 0/12 | 23.0 | 0/12 |
| 0 | | 0/12 | 22.8 | 0/12 |

Experimental Example 10

A plurality of groups each consisting of six male Swiss nude mice as test groups and twelve of them as the control group were implanted into the respective kidneys with human breast carcinoma MX-1. Each of the animals was administered subcutaneously with Y-6430 dissolved in physiological saline on the next day to the implantation, 5th day and 9th day. On 11th day, each of the weight of carcinoma was measured, and the tumor weight change (%) of the implanted carcinoma was calculated in accordance with the following scheme. The results are shown in Table 11.

$$\text{Tumor weight change (\%)} = \frac{\begin{pmatrix}\text{mean tumor weight of}\\\text{the test group on an}\\\text{evaluation day}\end{pmatrix} - \begin{pmatrix}\text{mean tumor weight of}\\\text{the test group on an}\\\text{implantation day}\end{pmatrix}}{\text{mean tumor weight of the test group on an implantation day}} \times 100$$

Table 11

| Dose (mg/kg, s.c.) | Proliferation Ratio (%) |
|---|---|
| 225 | -70 |
| 450 | -64 |
| 900 | -65 |

These data were based on the experiment conducted by National Cancer Institute, Bethesda, USA in accordance with the rëquest of the present inventors.

Experimental Example 11

Acute toxicity value when Y-6430 was administered intraperitoneally to male ddn mice was 1,500 mg/kg or more. Acute toxicity values when AH-44 carcinoma cells were implanted peritoneally and orally to Donryu rats were both 1,000 mg/kg or more.

From the foregoing experimental results, the compounds employed as the effective components of the composition of this invention were recognized as effective even by oral administration in the experimental tumor model including human carcinoma model, and their action mechanism was considered to be different from those of known anti-cancer preparations, thus their use as an anti-cancer agent of a novel type being expected. As to the safety of the compounds, no mutagenity was observed by Ames test and Rec assay, and no specific organ toxicity was observed even in 10-day preliminary toxicity, thus the anti-cancer compositions of this invention being expected as superior to known anti-cancer agents from

the viewpoint of safety.

From the foregoing, it is recognized that the compounds used as the effective components of the composition of this invention are useful as antitumor agents for various mammalian animals including men.

When these compounds are used antitumor agents, they can be incorporated with pharmaceutically acceptable additives such as carriers, excipients or diluents to be formulated into powders, granules, tablets, sugar-coated tablets, capsules, syrup, suppositories, topica or injections including for instillation.

The antitumor agents or this invention can be administered to mammalian animals including men orally or parenterally, but preferably by oral route. The amount to be administered may be such as effectively controlling the subject tumors, but it varies with the subject animals, kinds of tumors, administration routes, preparation forms, etc, but it is, in case of oral administration, 10-400 mg/kg/day, preferably 50-200 mg/kg/day, while, in case of injection, 1-10 mg/kg/day, preferably, 1-5 mg/kg/day, in 1-4 doses per day.

These antitumor compositions of this invention may be used together with any other anti-cancer drugs, immunoactivating agents or acceptable pharmaceuticals.

The following are examples of pharmaceutical preparations of the antitumor compositions of this invention, but it should be understood that this invention is not limited to them.

Preparation Example 1

Tablets consisting of:

| | |
|---|---|
| Y-6430 | 50.0 mg |
| finely powdered cellulose | 25.0 mg |
| maltose | 49.5 mg |
| starch | 40.0 mg |
| talc | 5.0 mg |
| magnesium stearate | 0.5 mg |

If desired, these tablets may be sugar-coated.

Preparation Example 2

Capsules composed of:

| | |
|---|---|
| Y-6430 | 50.0 mg |
| maltose | 50.0 mg |
| starch | 15.0 mg |
| talc | 5.0 mg |

Preparation Example 3

Granules consisting of:

| | |
|---|---|
| Y-6430 | 10% |
| maltose | 80% |
| starch | 10% |

Preparation Example 4

Granules consisting of:

| | |
|---|---|
| Y-6430 | 10% |
| maltose | 55% |
| finely powdered cellulose | 20% |
| starch | 15% |

Preparation Example 5

| | |
|---|---|
| Y-6430 | 50.0 mg |

| | |
|---|---|
| glucose | 100.0 mg |

are dissolved in distilled water to prepare 2 ml injection.

Preparation Example 6

Suppository consisting of:

| | |
|---|---|
| Y-6430 | 100 mg |
| Witepsol H15 | 950 mg |
| Witepsol E75 | 950 mg |

Witepsol is a tradename owned by Witten A.G. in West Germany.

Preparation Example 7

| | |
|---|---|
| Y-6430 | 2 g |
| ethyl parahydroxybenzoate | 0.025 g |
| propyl parahydroxybenzoate | 0.015 g |
| sodium lauryl sulfate | 1.5 g |
| propylene glycol | 12.0 g |
| stearyl alcohol | 22.0 g |
| white vaseline | 25.0 g |

are dissolved in distilled water to make 100.0 g of hydrophilic ointment.

The present invention has thus been described in the foregoing specification, experimental examples and preparation examples, but further alteration or modification may be possible so long as they are not against the spirit and scope of this invention.

Claims:

1. An antitumor composition characterized by containing at least one member selected from the group consisting of S-methylcarbamoyl-L-cysteine ethyl ester representable by the formula;

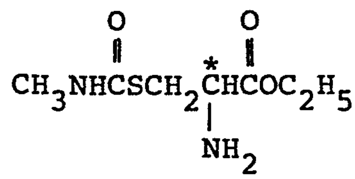 (I)

(wherein the carbon atom with asterisk at the α-position is L-configurated)

and its pharmaceutically acceptable acid addition salts.

2. The antitumor composition claimed in Claim 1, characterized by containing S-methylcarbamoyl-L-cysteine ethyl ester hydrochloride.

3. A method of therapy of tumors, which comprises administering, as an essential effective component, an effective amount of at least one member selected from the group consisting of S-methylcarbamoyl-L-cysteine ethyl ester representable by the formula;

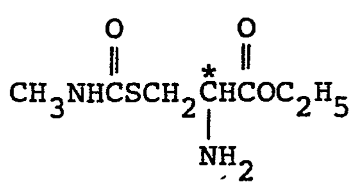 (I)

(wherein the carbon atom with asterisk at the α-position is L-configurated)

and its pharmaceutically acceptable acid addition salts.

4. The method of therapy of tumors claimed in Claim 3, which comprises administering, as an essential effective component, an effective amount of S-methylcarbamoyl-L-cysteine

ethyl ester hydrochloride.

5. A use, for the therapy of tumors, of S-methylcarbamoyl-L-cysteine ethyl ester representable by the formula;

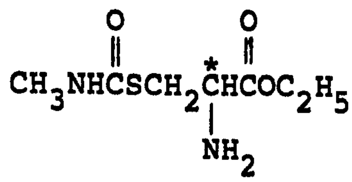

(I)

(wherein the carbon atom with asterisk at the α-position is L-configurated)

or of at least one compound of its acid addition salts.

6. Use of S-methylcarbamoyl-L-cysteine ethyl ester hydrochloride for the therapy of tumors claimed in Claim 5.

# INTERNATIONAL SEARCH REPORT

0139005

International Application No PCT JP ...

**I. CLASSIFICATION OF SUBJECT MATTER** (if several classification symbols apply, indicate all)

According to International Patent Classification (IPC) or to both National Classification and IPC

Int. $Cl^3$ A61K 31/27

**II. FIELDS SEARCHED**

Minimum Documentation Searched

| Classification System | Classification Symbols |
|---|---|
| IPC | A61K 31/27, C07C 155/02 |

Documentation Searched other than Minimum Documentation to the Extent that such Documents are Included in the Fields Searched

**III. DOCUMENTS CONSIDERED TO BE RELEVANT**

| Category* | Citation of Document, with indication, where appropriate, of the relevant passages | Relevant to Claim No |
|---|---|---|
| A | JP, A, 49-223 (Yoshitomi Pharmaceutical Industries, Ltd.) 5 January 1974 (05. 01. 74) (See Chem. Abstr. 80 121320q) | 1 - 6 |
| A | JP, A, 49-76824 (Yoshitomi Pharmaceutical Industries, Ltd.) 24 July 1974 (24. 07. 74) (See Chem. Abstr. 81 169850k) | 1 - 6 |
| A | JP, A, 49-135942 (Yoshitomi Pharmaceutical Industries, Ltd.) 27 December 1974 (27. 12. 74) (See Chem. Abstr. 82 156722r) | 1 - 6 |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 5, 1984 (05. 03. 84) | March 12, 1984 (12. 03. 84) |
| **International Searching Authority** | **Signature of Authorized Officer** |
| Japanese Patent Office | |